# EUROPÄISCHE PATENTANMELDUNG

(11) **EP 4 825 903 A1**
(43) Veröffentlichungstag der Anmeldung: **21.10.2026**
(21) Anmeldenummer: 25171194.1
(22) Anmeldetag: 17.04.2025
(51) Int. Cl.: C08K 5/00, C08K 5/12, C07C 67/02, C07C 69/003, C07C 69/604

(54) **WEICHMACHERZUSAMMENSETZUNG ENTHALTEND TRIPENTYLESTER DER 1,2,4-CYCLOHEXANTRIPROPIONSÄURE UND EINEN 1,2- ODER 1,4-DIALKYLCYCLOHEXANDICARBONSÄUREESTER**

(71) Anmelder: Evonik Oxeno GmbH & Co. KG, 45772 Marl (DE)
(72) Erfinder: GRASS, Michael, 45721 Haltern am See (DE); VAN EICKELS, Michael, 45657 Recklinghausen (DE); RUSEK, Monika, 45149 Essen (DE)
(74) Vertreter: Evonik Patent Association

(57) **Zusammenfassung**

Die vorliegende Erfindung betrifft eine Weichmacherzusammensetzung enthaltend eine Verbindung der Formel (1) 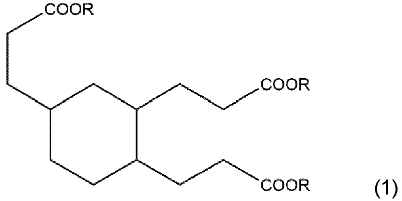 bei denen die beiden Alkylgruppen R jeweils 5 Kohlenstoffatome aufweisen, von denen 45 bis 97 Mol%, vorzugsweise 55 bis 97 Mol% n-Pentylreste, und mindestens einen 1,2- oder 1,4-Dialkylcyclohexandicarbonsäureester, wobei die beiden Alkylgruppen im 1,2- oder 1,4-Dialkylcyclohexandicarbonsäureester jeweils 8, 9 oder 10 Kohlenstoffatome aufweisen.

## Beschreibung

Gegenstand der Erfindung ist eine Weichmacherzusammensetzung enthaltend eine Verbindung der Formel (1) 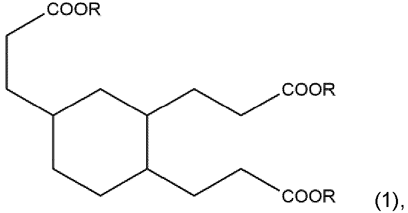 bei denen die beiden Alkylgruppen R jeweils 5 Kohlenstoffatome aufweisen, von denen 45 bis 97 Mol% n-Pentylreste sind, und mindestens einen 1,2- oder 1,4-Dialkylcyclohexandicarbonsäureester, wobei die beiden Alkylgruppen im 1,2- oder 1,4-Dialkylcyclohexandicarbonsäureester jeweils 8, 9 oder 10 Kohlenstoffatome aufweisen.

Dialkylester der 1,2- oder 1,4-Cyclohexandicarbonsäure sind seit einiger Zeit bekannt und auch im Markt etabliert. Relevante Vertreter sind die Diisononylester, die als Ester der 1,2-Cyclohexandicarbonsäure als DINCH (Diisononyl-1,2-cyclohexandicarbonsäureester) und als Ester der 1,4-Cyclohexandicarbonsäure als DINCD (Diisononyl-1,4-cyclohexandicarbonsäureester) kommerziell erhältlich sind. Die entsprechenden Di-2-ethylhexylester sind auch beschrieben.

Die Dialkylester der 1,2- oder 1,4-Cyclohexandicarbonsäure weisen in der Regel gute Viskositätseigenschaften und eine gute Kälteflexibilität auf, zeigen jedoch Schwächen bei der Weichmachereffizienz, der Gelierung und der Flüchtigkeit. Insofern wäre es wünschenswert, die Nachteile der Dialkylester der 1,2- oder 1,4-Cyclohexandicarbonsäure zu reduzieren.

Trialkylester der 1,2,4-Cyclohexantripropionsäure gemäß Formel (1), wobei die drei Alkylgruppen jeweils 5 Kohlenstoffatome aufweisen, sind auch grundsätzlich bekannt und beispielsweise in der EP 3 838 888 A1 beschrieben worden. Dort wurde auch erwähnt, dass diese Ester der 1,2,4-Cyclohexantripropionsäure als Weichmacher für Polymere eingesetzt werden können. Diese Produkte zeigen allerdings eine erhöhte Viskosität in Plastisolen und eine schlechtere Kälteflexibilität.

Der vorliegenden Erfindung lag nun die Aufgabe zugrunde, eine Weichmacherzusammensetzung bereitzustellen, die Dialkylester der 1,2- oder 1,4-Cyclohexandicarbonsäure enthält, aber bessere anwendungstechnische Eigenschaften aufweist als Weichmacherzusammensetzungen, die nur Dialkylester der 1,2- oder 1,4-Cyclohexandicarbonsäure enthalten, mit denen zwar in der Regel die Gelierung und Effizienz verbessert werden kann, oftmals aber Nachteile insbesondere bei der Flüchtigkeit resultieren.

Diese Aufgabe wird gelöst durch die Weichmacherzusammensetzung nach Anspruch 1. Gegenstand der Erfindung ist demnach eine Weichmacherzusammensetzung enthaltend eine Verbindung der Formel (1) 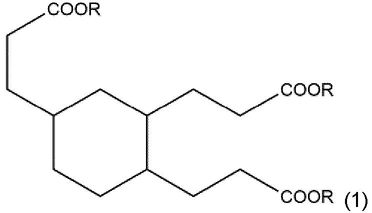 bei denen die beiden Alkylgruppen R jeweils 5 Kohlenstoffatome aufweisen, von denen 45 bis 97 Mol% n-Pentylreste sind, vorzugsweise 55 bis 97 Mol% n-Pentylreste sind und mindestens einen 1,2- oder 1,4-Dialkylcyclohexandicarbonsäureester, wobei die beiden Alkylgruppen im 1,2- oder 1,4-Dialkylcyclohexandicarbonsäureester jeweils 8, 9 oder 10 Kohlenstoffatome, vorzugsweise 8 oder 9 Kohlenstoffatome aufweisen. Bevorzugte Ausführungsformen der vorliegenden Erfindung sind in den Unteransprüchen angegeben. Die Reste R sind folglich Pentylreste mit einem gewissen Anteil am n-Pentyl-Isomer. Die Prozentangabe Mol% bezieht sich hier jeweils auf 100 Mol% aller Reste R der Verbindung nach Formel (1).

Im Rahmen der vorliegenden Erfindung werden die Begriffe "pentyl", "Pentylreste" oder ähnliches verwendet. Damit wird unterstrichen, dass die erfindungsgemäßen Ester verschiedene Pentyl-Isomere, insbesondere 2-Methylbutyl und/oder 3-Methylbutyl und/oder n-Pentyl, enthalten.

Die erfindungsgemäßen Verbindungen nach Formel (1) sind folglich Tripentylester der 1,2,4-Cyclohexantripropionsäure, in denen ein gewisser Anteil an einem oder mehreren Pentylresten vorhanden sind. Wichtigste Vertreter bei den Isomeren der Pentylgruppe sind die lineare n-Pentylgruppe sowie die einfach verzweigten 2-Methylbutyl- und 3-Methylbutylgruppen.

Die erfindungsgemäße Zusammensetzung umfasst Mischester der Tripentylester der 1,2,4-Cyclohexantripropionsäure, wobei mindestens 45 Mol% der Pentylreste, vorzugsweise mindestens 55 % der Pentylreste, besonders bevorzugt mindestens 75 Mol% und jeweils maximal 97 Mol% der Pentylreste im Mischester n-Pentylreste sind. Die Prozentangabe Mol% bezieht sich auch hier jeweils auf 100 Mol%, also auf alle Pentylreste im Mischester.

Die Summe aller vorhandenen Pentylreste im Mischester ergibt naturgemäß 100 Mol%. Die restlichen Pentylreste verteilen sich dann notwendigerweise auf 2-Methylbutyl- und/oder 3-Methylbutylreste. Es ist bevorzugt, den Anteil an 3-Methylbutylresten gering zu halten. Vorzugsweise sind in den Tripentylestern der 1,2,4-Cyclohexantripropionsäure weniger als 10 Mol%, vorzugsweise weniger als 5 Mol% der Pentylreste 3-Methylbutylreste. In einer besonders bevorzugten Ausführungsform enthält der 1,4-Dipentylcyclohexandicarbonsäureester kleiner 0,1 Mol% oder keine 3-Methylbutylreste. Die Prozentangabe Mol% bezieht sich auch hier jeweils auf 100 Mol%, also alle Pentylreste im Mischester.

Im Gegensatz dazu hat es sich als vorteilhaft erwiesen, wenn der Tripentylester der 1,2,4-Cyclohexantripropionsäure 2-Methylbutylreste enthält. Bevorzugt sind in den Tripentylestern der 1,2,4-Cyclohexantripropionsäure bis zu 55 Mol% der Pentylreste 2-Methylbutylreste, weiterhin bevorzugt bis zu 45 Mol% der Pentylreste 2-Methylbutylreste und besonders bevorzugt sind in den Tripentylestern der 1,2,4-Cyclohexantripropionsäure 3 bis 25 Mol% der Pentylreste 2-Methylbutylreste. Die Prozentangabe Mol% bezieht sich auch hier jeweils auf 100 Mol%, also alle Pentylreste im Mischester. Diese Mischungen sind auch deshalb bevorzugt, weil großtechnisch verfügbare Pentanole in der Regel Mischungen von Pentanol und 2-Methylbutanol sind, die einen Anteil von > 90 Mol% n-Pentanol und < 10 Mol% 2-Methylbutanol aufweisen.

Neben den Tripentylestern der 1,2,4-Cyclohexantripropionsäure enthält die erfindungsgemäße Weichmacherzusammensetzung 1,2- oder 1,4-Dialkylcyclohexandicarbonsäureester, wobei die beiden Alkylgruppen jeweils unabhängig voneinander 8, 9 oder 10 Kohlenstoffatome, vorzugsweise 8 oder 9 Kohlenstoffatome aufweisen.

Die entsprechenden 1,4-Dialkylcyclohexandicarbonsäureester können durch die nachfolgende Markush-Formel beschrieben werden: 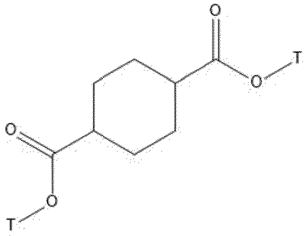 wobei die beiden Reste T jeweils unabhängig voneinander Alkylgruppen mit 8, 9 oder 10 Kohlenstoffatomen, vorzugsweise 8 oder 9 Kohlenstoffatomen sind.

Die beiden Alkylgruppen können dabei jeweils unabhängig voneinander aus der Gruppe, bestehend aus einer normalen Octylgruppe, einer Isooctylgruppe, einer 2-Ethylhexylgruppe, einer normalen Nonylgruppe, einer Isononylgruppe, einer n-Decylgruppe, einer Isodecylgruppe oder einer 2-Propylheptylgruppe, vorzugsweise aus der Gruppe, bestehend aus einer normalen Octylgruppe, einer Isooctylgruppe, einer 2-Ethylhexylgruppe, einer normalen Nonylgruppe und einer Isononylgruppe ausgewählt werden. Unter einer Isooctylgruppe wird im Sinne der vorliegenden Erfindung eine Mischung aus linearen und verzweigten primären C8-Akylgruppen verstanden. Analog versteht man unter einer Isononylgruppe und einer Isodecylgruppe jeweils eine Mischung aus linearen und verzweigten primären C9-Alkylgruppen bzw. C10-Alkylgruppen.

In einer bevorzugten Ausführungsform der vorliegenden Erfindung weisen die beiden Alkylgruppen des 1,4-Dialkylcyclohexandicarbonsäureesters jeweils die gleiche Anzahl an Kohlenstoffatomen auf. Das bedeutet, dass beide Alkylgruppen 8, dass beide Alkylgruppen 9 Kohlenstoffatome oder dass beide Alkylgruppen 10 Kohlenstoffatome aufweisen, aber nicht, dass beide Alkylgruppen hinsichtlich der jeweiligen Isomere immer identisch sein müssen.

Es ist jedoch besonders bevorzugt, dass der 1,4-Dialkylcyclohexandicarbonsäureester in der erfindungsgemäßen Zusammensetzung 1,4-Di-isononyl-cyclohexandicarbonsäureester (1,4-DINCH bzw. DINCD) oder 1,4-Di-2-ethylhexyl-cyclohexandicarbonsäureester (1,4-DEHCH) ist.

Die Herstellung der erfindungsgemäßen 1,4-Dialkylcyclohexandicarbonsäureester kann über direkte Veresterung der 1,4-Cyclohexandicarbonsäure mit einem Alkohol mit 8, 9 oder 10 Kohlenstoffatomen, vorzugsweise 8 oder 9 Kohlenstoffatomen erfolgen. Der Alkohol wird dabei vorzugsweise im Überschuss eingesetzt, d. h. in einer Menge von mehr als 2 Mol pro Mol Säurekomponente. Die direkte Veresterung wird weiterhin bevorzugt an einem sauren Katalysator durchgeführt, beispielsweise anorganische, organische oder Lewis-Säuren. Entsprechende Verfahren sind dem Fachmann bekannt.

Die Herstellung der erfindungsgemäßen 1,4-Dialkylcyclohexandicarbonsäureester kann auch über eine Umesterung erfolgen, wobei ein 1,4-Dialkylcyclohexandicarbonsäureester mit Alkylgruppen mit weniger als 8 Kohlenstoffatomen eingesetzt und mit einem Alkohol mit 8, 9 oder 10 Kohlenstoffatomen, vorzugsweise mit 8 oder 9 Kohlenstoffatomen umgeestert wird. Auch hier kann der Alkohol mit 8, 9 oder 10 Kohlenstoffatomen, vorzugsweise mit 8 oder 9 Kohlenstoffatomen im Überschuss eingesetzt werden. Auch die Umesterung ist ein dem Fachmann bekanntes Verfahren.

Vorzugsweise erfolgt die Herstellung aber über die Veresterung von Terephthalsäure mit einem Alkohol mit 8, 9 oder 10 Kohlenstoffatomen, vorzugsweise mit 8 oder 9 Kohlenstoffatomen oder durch Umesterung eines Terephthalsäureesters, dessen Alkylgruppe weniger als 8 Kohlenstoffatome aufweist, vorzugsweise Dimethylterephthalat. Es entstehen dabei Dialkylterephthalsäureester, deren Alkylgruppen 8, 9 oder 10 Kohlenstoffatome, vorzugsweise 8 oder 9 Kohlenstoffatome aufweisen. Anschließend werden die so hergestellten Dialkylterephthalsäureester kernhydriert, um die gewünschten 1,4-Dialkylcyclohexandicarbonsäureester zu erhalten. Verfahren zur Kernhydrierung sind in der Literatur beschrieben und dem Fachmann bekannt.

Die entsprechenden 1,2-Dialkylcyclohexandicarbonsäureester können durch die nachfolgende Markush-Formel beschrieben werden: 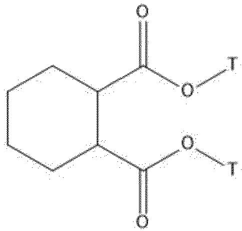 wobei die beiden Reste T jeweils unabhängig voneinander Alkylgruppen mit 8, 9 oder 10 Kohlenstoffatomen, vorzugsweise mit 8 oder 9 Kohlenstoffatomen sind.

Die beiden Alkylgruppen können dabei jeweils unabhängig voneinander aus der Gruppe, bestehend aus einer normalen Octylgruppe, einer Isooctylgruppe, einer 2-Ethylhexylgruppe, einer normalen Nonylgruppe, einer Isononylgruppe, einer n-Decylgruppe, einer Isodecylgruppe oder einer 2-Propylheptylgruppe, vorzugsweise aus der Gruppe, bestehend aus einer normalen Octylgruppe, einer Isooctylgruppe, einer 2-Ethylhexylgruppe, einer normalen Nonylgruppe und einer Isononylgruppe ausgewählt werden. Unter einer Isooctylgruppe wird im Sinne der vorliegenden Erfindung eine Mischung aus linearen und verzweigten primären C8-Alkylgruppen verstanden. Analog versteht man unter einer Isononylgruppe und einer Isodecylgruppe jeweils eine Mischung aus linearen und verzweigten primären C9-Alkylgruppen bzw. C10-Alkylgruppen.

In einer bevorzugten Ausführungsform der vorliegenden Erfindung weisen die beiden Alkylgruppen des 1,2-Dialkylcyclohexandicarbonsäureesters jeweils die gleiche Anzahl an Kohlenstoffatomen auf. Das bedeutet, dass beide Alkylgruppen 8, beide Alkylgruppen 9 oder beide Alkylgruppen 10 Kohlenstoffatome aufweisen, aber nicht, dass beide Alkylgruppen hinsichtlich der jeweiligen Isomere immer identisch sein müssen.

Es ist jedoch besonders bevorzugt, dass der 1,2-Dialkylcyclohexandicarbonsäureester in der erfindungsgemäßen Zusammensetzung 1,2-Di-isononyl-cyclohexandicarbonsäureester (DINCH) oder 1,2-Di-2-ethylhexyl-cyclohexandicarbonsäureester (DEHCH) ist.

Die Herstellung der erfindungsgemäßen 1,2-Dialkylcyclohexandicarbonsäureester kann über direkte Veresterung der 1,2-Cyclohexandicarbonsäure oder des entsprechenden Anhydrids mit einem Alkohol mit 8, 9 oder 10 Kohlenstoffatomen, vorzugsweise mit 8 oder 9 Kohlenstoffatomen erfolgen. Der Alkohol wird dabei vorzugsweise im Überschuss eingesetzt, d. h. in einer Menge von mehr als 2 Mol pro Mol Säurekomponente. Die direkte Veresterung wird weiterhin bevorzugt an einem sauren Katalysator durchgeführt, beispielsweise anorganische, organische oder Lewis-Säuren. Alternativ können die erfindungsgemäßen 1,2-Dialkylcyclohexandicarbonsäureester auch über eine Reaktionssequenz, die eine Diels-Alder-Reaktion aus Butadien und Maleinsäureanhydrid einschließt, hergestellt werden. Das so entstehende sog. Tetrahydrophthalsäureanhydrid wird anschließend mit Alkoholen verestert und die Doppelbindung hydriert. Entsprechende Verfahren sind dem Fachmann bekannt.

Die Herstellung der erfindungsgemäßen 1,2-Dialkylcyclohexandicarbonsäureester kann auch über eine Umesterung erfolgen, wobei ein 1,2-Dialkylcyclohexandicarbonsäureester mit Alkylgruppen mit weniger als 8 Kohlenstoffatomen eingesetzt und mit einem Alkohol mit 8, 9 oder 10 Kohlenstoffatomen, vorzugsweise mit 8 oder 9 Kohlenstoffatomen umgeestert wird. Auch hier kann der Alkohol mit 8, 9 oder 10 Kohlenstoffatomen, vorzugsweise mit 8 oder 9 Kohlenstoffatomen im Überschuss eingesetzt werden. Entsprechende Verfahren sind dem Fachmann ebenfalls bekannt.

Vorzugsweise erfolgt die Herstellung aber über die Veresterung von Phthalsäure, besonders bevorzugt mit Phthalsäureanhydrid mit einem Alkohol mit 8, 9 oder 10 Kohlenstoffatomen, vorzugsweise mit 8 oder 9 Kohlenstoffatomen oder durch Umesterung eines Phthalsäureesters, dessen Alkylgruppe weniger als 8 Kohlenstoffatome aufweist. Es entstehen dabei Dialkylphthalsäureester, deren Alkylgruppen 8, 9 oder 10 Kohlenstoffatome, vorzugsweise 8 oder 9 Kohlenstoffatome aufweisen. Anschließend werden die so hergestellten Dialkylphthalsäureester kernhydriert, um die gewünschten 1,2-Dialkylcyclohexandicarbonsäureester zu erhalten. Verfahren zur Kernhydrierung sind in der Literatur beschrieben und dem Fachmann bekannt.

Die beiden Substanzen können in unterschiedlichen Mengen in der erfindungsgemäßen Weichmacherzusammensetzung vorhanden sein. Es sollte klar sein, dass die beiden Substanzen in einer Menge vorhanden sein müssen, bei der sie eine Wirkung entfalten, d. h. wo eine weichmachende Wirkung eintritt. In einer bevorzugten Ausführungsform der vorliegenden Erfindung liegen die Verbindung nach Formel (1) und der 1,2- oder 1,4-Dialkylcyclohexandicarbonsäureester in einem Gewichtsverhältnis (Verbindung nach Formel (1) : 1,2- oder 1,4-Dialkylcyclohexandicarbonsäureester) von 1 : 99 bis 99 : 1, vorzugsweise 10 : 90 bis 90 : 10, besonders bevorzugt 20 : 80 bis 80 : 20 in der Weichmacherzusammensetzung vor. In einer ganz besonders bevorzugten Ausführung der vorliegenden Erfindung liegen die Verbindung nach Formel (1) und der 1,2- oder 1,4-Dialkylcyclohexandicarbonsäureester in einem Gewichtsverhältnis (Verbindung nach Formel (1) : 1,2- oder 1,4-Dialkylcyclohexandicarbonsäureester) von 40 : 60 bis 10 : 90 in der Weichmacherzusammensetzung vor. Durch gezielte Einstellung der Mischungsverhältnisse können anwendungsspezifisch bestimmte Eigenschaften gezielt verbessert werden, ohne zu große Kompromisse in den anderen Eigenschaften eingehen zu müssen.

Die erfindungsgemäße Weichmacherzusammensetzung der vorliegenden Erfindung kann zusätzlich ein epoxidiertes Öl oder einen epoxidierten Alkylester einer Fettsäure enthalten. Epoxidierte Öle oder entsprechende Ester können die thermische Stabilität und die mechanischen Eigenschaften verbessern und je nach ihrem Anteil auch weichmachende Eigenschaften aufweisen.

Das epoxidierte Öl kann aus der Gruppe bestehend aus epoxidiertem Sojabohnenöl, epoxidiertem Rizinusöl, epoxidiertem Leinöl, epoxidiertem Palmöl, epoxidiertem Tallöl und Mischungen davon ausgewählt werden. Bevorzugt werden epoxidiertes Sojabohnenöl oder epoxidiertes Leinöl in der erfindungsgemäßen Weichmacherzusammensetzung eingesetzt. Besonders bevorzugt ist epoxidiertes Sojabohnenöl, das auch unter dem Akronym ESBO bekannt ist.

Die epoxidierten Fettsäureester können durch Umesterung der o.g. epoxidierten Öle mit Alkoholen im Bereich von 1 bis 10 Kohlenstoffatomen, bevorzugt 4 bis 9 Kohlenstoffatomen, hergestellt werden. Alternativ kann erst das natürliche Öl umgeestert und die Doppelbindungen der Fettsäure anschließend epoxidiert werden.

Das epoxidierte Öl oder die entsprechenden epoxidierten Fettsäurealkylester können in einer Menge von 1 bis 150 Gewichtsteilen bezogen auf 100 Gewichtsteile der Gesamtsumme aus der Verbindung gemäß Formel (1) und dem 1,2- oder 1,4-Dialkylcyclohexandicarbonsäureester eingesetzt werden. Das epoxidierte Öl oder die epoxidierten Fettsäureester können auch in Mengen von 2 bis 125 Gewichtsteilen, 5 bis 100 Gewichtsteilen, 10 bis 80 Gewichtsteilen oder 20 bis 70 Gewichtsteilen jeweils bezogen auf 100 Gewichtsteile der Gesamtsumme aus der Verbindung gemäß Formel (1) und dem 1,2-oder 1,4-Dialkylcyclohexandicarbonsäureester in der Weichmacherzusammensetzung enthalten sein.

Die erfindungsgemäße Weichmacherzusammensetzung kann zusätzlich noch mindestens einen weiteren Weichmacher enthalten, der aus der Gruppe, bestehend aus Adipaten, Benzoaten, beispielsweise Monobenzoaten oder Glycoldibenzoaten, chlorierten Kohlenwasserstoffen (sog. Chlorparaffinen), Citraten, epoxidierten Fettsäureestern, epoxidierten Pflanzenölen, epoxidierten acylierten Glyceriden, Isosorbidestern, Furandicarboxylaten, Phosphaten, Succinaten, Sulfonamiden, Alkylsulfonsäureestern des Phenols, Phthalaten, Terephthalaten, Isophthalaten, Cyclohexandicarboxylaten mit Ausnahme der bereits vorhandenen Ester, Trimellitaten sowie Polyolestern und oligomeren oder polymeren Estern auf Basis von Adipin-, Bernstein- oder Sebacinsäure, ausgewählt wird.

In einer bevorzugten Ausführungsform der vorliegenden Erfindung enthält die Weichmacherzusammensetzung mindestens einen weiteren Weichmacher, der aus der Gruppe, bestehend aus Alkylbenzoaten, Alkylsulfonsäureestern des Phenols, Dialkyladipaten, Glycerinestern, C4-bis C6- Alkansäureestern von Polyolen, acetylierten oder nicht acetylierten Citronensäuretrialkylestern, Glykoldibenzoaten, Trialkylestern der Trimellitsäure, Dialkylterephthalaten, Dialkylphthalaten, Dialkylisophthalaten, Estern der Furandicarbonsäure, Dialkanoylestern von Dianhydrohexitolen (z.B. Isosorbid), epoxidierten Fettsäurealkylestern, Polymerweichmachern, beispielsweise der Polyadipate sowie den Dialkylestern der 1,2- oder 1,4-Cyclohexandicarbonsäure mit Ausnahme der bereits vorhandenen Ester mit 8 oder 9 Kohlenstoffatomen und Dialkylestern der 1,3-Cyclohexandicarbonsäure, ausgewählt wird.

In einer weiterhin bevorzugten Ausführungsform wird der mindestens eine weitere Weichmacher, der in der erfindungsgemäßen Weichmacherzusammensetzung enthalten sein kann, aus der Gruppe, bestehend aus C8- bis C13-Alkylbenzoaten, C4- bis C10-Dialkyladipaten, Pentaerythrit-tetravalerat, acetylierten oder nicht acetylierten Citronensäuretrialkylestern mit C4 bis C9-Alkylgruppen, C4- bis C10-Trialkyltrimellitaten, C4- bis C9-Dialkylterephthalaten, C4- bis C13-Dialkylphthalaten, insbesondere C9-bis C13-Dialkylphthalaten und C4- bis C10-Dialkylestern der 1,3-Cyclohexandicarbonsäure, sowie den C4-C7-Dialkylestern der 1,2- bzw. 1,4-Cyclohexandicarbonsäure ausgewählt.

Besonders bevorzugt werden von den Citraten Butyl- oder Pentyl- oder höhere Citrate eingesetzt, die eine Acetylgruppe aufweisen können. Darunter fallen Tributylcitrat, Tripentylcitrat, Tri-n-hexylcitrat, Tri-2-ethylhexylcitrat, Triisononylcitrat, Acetyltributylcitrat und Acetyltripentylcitrat. Ebenso gehören zu dieser Gruppe auch acetylierte oder nicht acetylierte Ester der Citronensäure, bei denen die Alkylketten unterschiedliche Kettenlägen aufweisen können, wie zum Beispiel C4/C8 oder C5/C9. Von den C4- bis C10-Dialkyladipaten sind Diethylhexyladipat (DEHA) und Diisononyladipat (DINA) bevorzugt. Von den C4- bis C9-Dialkylterephthalaten sind Dibutylterephthalat (DBT), Dipentylterephthalat (DPT) und Diethylhexylterephthalat (DEHT bzw. DOTP) bevorzugt. Von den C4- bis C10-Trialkyltrimellitaten sind Triethylhexyltrimellitat (TEHTM bzw. TOTM), und Triisononyltrimellitat (TINTM) sowie der Trimellitsäureester mit linearen C8- und C10-Alkoholen (T810TM) bevorzugt.

Ein weiterer Gegenstand der vorliegenden Erfindung ist eine Kunststoffzusammensetzung, die einen Kunststoff und die Weichmacherzusammensetzung, umfassend die Verbindung der Formel (1) und mindestens einen 1,2- oder 1,4-Dialkylcyclohexandicarbonsäureester, wobei die beiden Alkylgruppen jeweils 8, 9 oder 10 Kohlenstoffatome, vorzugsweise jeweils 8 oder 9 Kohlenstoffatome aufweisen, enthält. Die Kunststoffzusammensetzung kann zudem das epoxidierte Öl und /oder ein epoxidierten Fettsäurealkylester und/oder mindestens einen zusätzlichen Weichmacher aus der oben genannten Liste enthalten.

Geeignete Kunststoffe sind polymere Substanzen, die vorzugsweise aus der Gruppe, bestehend aus PVC (Polyvinylchlorid), Homo- oder Copolymere auf Basis von Ethylen, Propylen, Butadien, Vinylacetat, Glycidylacrylat, Glycidylmethacrylat, Ethylacrylat, Butylacrylat oder Methacrylat mit Alkoxyresten von verzweigten oder unverzweigten Alkoholen mit einem bis zehn Kohlenstoffatom(en), Acrylnitril oder cyclischen Olefinen, Polyvinylidenchlorid (PVDC), Polyacrylaten, insbesondere Polymethylmethacrylat (PMMA), Polyalkylmethacrylat (PAMA), Polyharnstoffe, silylierte Polymere, Fluorpolymere, insbesondere Polyvinylidenfluorid (PVDF), Polytetrafluorethylen (PTFE), Polyvinylacetat (PVAc), Polyvinylalkohol (PVA), Polyvinylacetale, insbesondere Polyvinylbutyral (PVB), Polystyrolpolymere, insbesondere Polystyrol (PS), expandierbares Polystyrol (EPS), Acrylonitril-Styrol-Acrylat (ASA), Styrolacrylonitril (SAN), Acrylonitril-Butadien-Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxybutyral (PHB), Polyhydroxyvaleriansäure (PHV), Polyester, Stärke, Cellulose und Cellulose-Derivate, insbesondere Nitrocellulose (NC), Ethylcellulose (EC), Celluloseacetat (CA), Cellulose-Acetat/Butyrat (CAB), Gummi und Silikonen, ausgewählt werden.

In einer bevorzugten Ausführungsform ist der Kunststoff ein Polymer, ausgewählt aus der Gruppe bestehend aus Polyvinylchlorid (PVC), Polyalkylmethacrylat (PAMA), Polyvinylbutyral (PVB), Polyurethan, Polysulfide, Polymilchsäure (PLA), Polyhydroxybutyral (PHB), Nitrocellulose und Co-Polymeren von Vinylchlorid mit Vinylacetat oder mit Butylacrylat. Besonders bevorzugt ist hiervon PVC als Kunststoff in der erfindungsgemäßen Kunststoffzusammensetzung.

Die Menge der erfindungsgemäßen Weichmacherzusammensetzung in der Kunststoffzusammensetzung beträgt vorzugsweise 5 bis 150 Gewichtsteile, bevorzugt 10 bis 120 Gewichtsteile, besonders bevorzugt 15 bis 110 Gewichtsteile und ganz besonders bevorzugt 20 bis 100 Gewichtsteile pro 100 Gewichtsteile des Kunststoffs.

Die Kunststoffzusammensetzung kann neben den erwähnten Inhaltsstoffen zusätzliche Additive enthalten. Beispiele für zusätzliche Additive sind rheologische Additive, mit denen die Viskosität der Kunststoffzusammensetzung verringert werden kann. Beispiele für bekannte rheologische Additive sind die unter den Handelsnamen VISCOBYK^{®}-5120, VISCOBYK^{®}-5130 und VISCOBYK^{®}-4041 erhältlichen Produkte. Die Additive können in einem Anteil von 1 bis 12, bevorzugt 2 bis 10 Gewichtsteilen pro 100 Gewichtsteilen PVC in der Kunststoffzusammensetzung enthalten sein.

Darüber hinaus kann die Kunststoffzusammensetzung einen oder mehrere Thermostabilisator(en) enthalten. Geeignete Thermostabilisatoren sind Bleisalze, Organozinnverbindungen, Barium/Zinkverbindungen, Cadmiumverbindungen oder Zinkverbindungen, Calcium/Zink-Stabilisatoren und organisch-basierte Stabilisatoren (sog. OBS). Bevorzugte Thermostabilisatoren sind Barium/Zinkverbindungen, Calcium/Zink-Stabilisatoren und organisch-basierte Stabilisatoren (sog. OBS). Der Anteil des oder der Stabilisatoren in der Kunststoffzusammensetzung beträgt vorzugsweise 1 bis 6 Gewichtsteile pro 100 Gewichtsteilen PVC.

Als weitere Additive können darüber hinaus auch Füllstoffe, Pigmente, Treibmittel, UV-Stabilisatoren und Gleitmittel in der Kunststoffzusammensetzung enthalten sein.

Die erfindungsgemäße Kunststoffzusammensetzung ist vorzugsweise Bestandteil eines Klebstoffs, einer Dichtungsmasse, einer Beschichtungsmasse, eines Lacks, einer Farbe, eines Plastisols, eines Dryblends, eines Schaums, eines Kunstleders, eines Fußbodenbelags, insbesondere dessen Deck- oder Schaumschicht, einer Dachbahn, eines Unterbodenschutzes, einer Gewebebeschichtung, eines Kabels, einer Drahtisolierung, eines Schlauchs, eines Extrusionsartikels, einer Folie, eines Gegenstands im Automobilinnenbereich, einer Tapete, einer Tinte, eines Spielzeugs, einer Kontaktfolie, einer Lebensmittelverpackung oder eines medizinischen Artikels, insbesondere eines Schlauchs oder eines Blutbeutels.

Gegenstand der vorliegenden Erfindung ist deshalb auch die Verwendung der Kunststoffzusammensetzung in Klebstoffen, Dichtungsmassen, Beschichtungsmassen, Lacken, Farben, Plastisolen, Schäumen, Kunstleder, Fußbodenbelägen, insbesondere Deck- und Schaumschicht, Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, insbesondere in Schläuchen oder Blutbeuteln.

Die vorliegende Erfindung wird nachfolgend anhand von Beispielen illustriert. Die folgenden Beispiele sollen die Erfindung erläutern, ohne deren Anwendungsbreite, die sich aus der Beschreibung und den Patentansprüchen ergibt, einzuschränken.

### Beispiel 1 - Herstellung der verschiedenen Pentylester der 1,2,4-Cyclohexantripropionsäure

Trimethyl-cyclohexan-1,2,4-tripropionat (Me-Tc) wurde gemäß Beispiel 0 der EP 3 838 888 A1 hergestellt und in einer Umesterungsapparatur mit 2-Methylbutanol (Mb) und n-Pentanol (Pe) (molares Verhältnis s. Tabelle 1) zum Produkt MbPe-Tc umgeestert. Die Umesterungsapparatur bestand aus einem Glaskolben, der mit einem Rührer, Temperaturfühler, einer Destillationskolonne, Vakuumteiler und Destillationsaufbau ausgestattet war.

Zunächst wurden die in Tabelle 1 aufgeführten Mischungen aus n-Pentanol und 2-Methylbutanol und die in Tabelle 1 angegebene Menge an Me-Tc in der Umesterungsapparatur vorgelegt. Im Anschluss wurde die gesamte Apparatur mit Stickstoff gespült und danach Tetra(n-butyl)titanat (TNBT) als Katalysator hinzugegeben (0,15 mol% bezogen auf Me-Tc). Danach wurde die Reaktion durch Aufheizen gestartet. Die Reaktionstemperatur betrug dabei 130 bis 240°C, d. h. sie stieg während der Reaktion an. Methanol, dass bei der Umesterung entstand, wurde über die Kolonne und Destillationsapparatur aus dem System entfernt (65°C Kopftemperatur). Die C5-Alkohole, welche in der Kolonne vom Methanol getrennt wurden, wurden als Rückfluss wieder in das Reaktionsgemisch zurückgeführt. Als kein Destillatanfall mehr zu beobachten war, wurde der Reaktionsfortschritt über eine GC-Analyse kontrolliert. Die Reaktion wurde beendet als der Tripentylester >98,8 GC-Flächen% ausmachte, bezogen auf die Summe der Flächen des Edukts, Mono-, Di- und Triesters.

Im Anschluss an die Reaktion wurde der überschüssige Alkohol bei 160 °C unter Vakuum abdestilliert. Danach wurde die Säurezahl der Reaktionsmischung bestimmt und diese dann durch die Zugabe der 3-fachen stöchiometrischen Menge an 10%-iger NaOH neutralisiert. Als nächstes wurde die Mischung bei 160 °C mit Stickstoff für 2 h gestrippt. Im letzten Schritt wurde das Produkt über einen 0,5 µm PTFE-Filter und Perlite als Filterhilfsmittel klar filtriert.

**Tabelle 1: Pentanolmischungen für die Herstellung der Cyclohexan-1,2,4-Tripropionsäure-tripentylester**

| Mischung (molares Verhältnis von an Pentanol (Pe) und 2-Methylbutanol (Mb)) | Menge Me-Tc / g (mol) | Menge n-Pentanol / g (mol) | Menge 2-Methylbutanol / g (mol) |
|---|---|---|---|
| A) (Pe/Mb = 50/50) | 1543 (4,5) | 743 (8,4) | 743 (8,4) |
| B) (Pe/Mb = 65/35) | 1710 (5,0) | 1071 (12,2) | 577 (6,6) |
| C) (Pe/Mb = 93/7) | 1883 (5,5) | 1690 (19,2) | 127 (1,4) |

### Beispiel 2 - Herstellung von Plastisolen

Es wurden PVC-Plastisole hergestellt, wie sie beispielsweise zur Fertigung von Deckstrichfilmen für Fußbodenbeläge verwendet werden. Die Angaben in der Plastisolrezeptur sind jeweils in Gewichtsteilen (phr). Die Rezepturen der Polymerzusammensetzung sind in Tabelle 2-4 aufgelistet.

**Tabelle 2: Übersicht über die hergestellten Plastisole mit DINCH bzw. DINCD und MbPe-Tc (50:50).**

| Plastisol | 1 | 2 | 3* | 4* | 5* | 6 | 7* | 8* | 9* | 10* |
|---|---|---|---|---|---|---|---|---|---|---|
| | phr | phr | phr | phr | phr | phr | phr | phr | phr | phr |
| PVC (Vestolit^{®} P 1430 K 70 Ultra; Fa. Vestolit) | 100 | 100 | 100 | 100 | 100 | 100 | 100 | 100 | 100 | 100 |
| ELATUR^{®} CH, (Di-isononyl-1,2-cyclohexanoat, Fa. Evonik Oxeno & Co. KG) | 50 | 37,5 | 33,33 | 25 | 16,67 | | | | | |
| ELATUR^{®} DINCD, (Di-isononyl-1,4-cyclohexanoat, Fa. Evonik Oxeno & Co. KG) | | | | | | 50 | 37,5 | 33,33 | 25 | 16,67 |
| A) MbPe-Tc (50:50) (2-Methylbutyl-n-pentyl-3,3',3"-(cyclohexan-1,2,4-triyl)proprionat) | | 12,5 | 16,67 | 25 | 33,33 | | 12,5 | 16,67 | 25 | 33,33 |
| epoxidiertes Sojabohnenöl als Co-Stabilisator (Edenol^{®} D81, Fa. Emery) | 3 | 3 | 3 | 3 | 3 | 3 | 3 | 3 | 3 | 3 |
| Thermostabilisator auf Ca/Zn-Basis (Reagens MBL 197/9 PF) | 2 | 2 | 2 | 2 | 2 | 2 | 2 | 2 | 2 | 2 |

| | | | | | | | | | | |
|---|---|---|---|---|---|---|---|---|---|---|
| * = erfindungsgemäße Zusammensetzung phr = parts per hundred parts resin | | | | | | | | | | |

**Tabelle 3: Übersicht über die hergestellten Plastisole mit DINCH bzw. DINCD und MbPe-Tc (35:65).**

| Plastisol | 11* | 12* | 13* | 14* | 15* | 16* | 17* | 18* |
|---|---|---|---|---|---|---|---|---|
| | phr | phr | phr | phr | phr | phr | phr | phr |
| PVC (Vestolit^{®} P 1430 K 70 Ultra; Fa. Vestolit) | 100 | 100 | 100 | 100 | 100 | 100 | 100 | 100 |
| ELATUR^{®} CH, (Di-isononyl-1,2-cyclohexanoat, Fa. Evonik Oxeno & Co. KG) | 37,5 | 33,33 | 25 | 16,67 | | | | |
| ELATUR^{®} DINCD, (Di-isononyl-1,4-cyclohexanoat, Fa. Evonik Oxeno & Co. KG) | | | | | 37,5 | 33,33 | 25 | 16,67 |
| B) MbPe-Tc (35:65) (2-Methylbutyl-n-pentyl-3,3',3"-(cyclohexan-1,2,4-triyl)proprionat) | 12,5 | 16,67 | 25 | 33,33 | 12,5 | 16,67 | 25 | 33,33 |
| epoxidiertes Sojabohnenöl als Co-Stabilisator (Edenol^{®} D81, Fa. Emery) | 3 | 3 | 3 | 3 | 3 | 3 | 3 | 3 |
| Thermostabilisator auf Ca/Zn-Basis (Reagens MBL 197/9 PF) | 2 | 2 | 2 | 2 | 2 | 2 | 2 | 2 |

| | | | | | | | | |
|---|---|---|---|---|---|---|---|---|
| * = erfindungsgemäße Zusammensetzung phr = parts per hundred parts resin | | | | | | | | |

**Tabelle 4: Übersicht über die hergestellten Plastisole mit DINCH bzw. DINCD und MbPe-Tc (7:93).**

| Plastisol | 19* | 20* | 21* | 22* | 23* | 24* | 25* | 26* |
|---|---|---|---|---|---|---|---|---|
| | phr | phr | phr | phr | phr | phr | phr | phr |
| PVC (Vestolit^{®} P 1430 K 70 Ultra; Fa. Vestolit) | 100 | 100 | 100 | 100 | 100 | 100 | 100 | 100 |
| ELATUR^{®} CH, (Di-isononyl-1,2-cyclohexanoat, Fa. Evonik Oxeno & Co. KG) | 37,5 | 33,33 | 25 | 16,67 | | | | |
| ELATUR^{®} DINCD, (Di-isononyl-1,4-cyclohexanoat, Fa. Evonik Oxeno & Co. KG) | | | | | 37,5 | 33,33 | 25 | 16,67 |
| C) MbPe-Tc (7:93) (2-Methylbutyl-n-pentyl-3,3',3"-(cyclohexan-1,2,4-triyl)proprionat) | 12,5 | 16,67 | 25 | 33,33 | 12,5 | 16,67 | 25 | 33,33 |
| epoxidiertes Sojabohnenöl als Co-Stabilisator (Edenol^{®} D81, Fa. Emery) | 3 | 3 | 3 | 3 | 3 | 3 | 3 | 3 |
| Thermostabilisator auf Ca/Zn-Basis (Reagens MBL 197/9 PF) | 2 | 2 | 2 | 2 | 2 | 2 | 2 | 2 |

| | | | | | | | | |
|---|---|---|---|---|---|---|---|---|
| * = erfindungsgemäße Zusammensetzung phr = parts per hundred parts resin | | | | | | | | |

### Beispiel 3 - Gelierung der Plastisole

Die Untersuchung des Gelierverhaltens der Plastisole aus Beispiel 2 wurde mittels Physica MCR 101 im Oszillationsmodus mit einem Platte-Platte Messsystem (PP25), welches schubspannungsgesteuert betrieben wurde, vorgenommen. Eine zusätzliche Temperierhaube wurde an das Gerät angeschlossen, um eine homogene Wärmeverteilung und eine gleichmäßige Probentemperatur zu erreichen.

### Folgende Parameter wurden eingestellt:

| | |
|---|---|
| Modus: | Temperatur-Gradient |
| Start-Temperatur: | 25 °C |
| End-Temperatur: | 180 °C |
| Heiz/Kühlrate: | 5 °C/min |
| Oszillations-Frequenz: | 4 bis 0,1 Hz Rampe logarithmisch |
| Kreisfrequenz Omega: | 10 s⁻¹ |
| Anzahl Messpunkte: | 63 |
| Messpunktdauer: | 0,5 min |

| | |
|---|---|
| Automatische Spaltnachführung F: | 0 N |
| Konstante Messpunktdauer | |
| Spaltweite | 0,5 mm |

### Durchführung der Messung

Auf die untere Messsystemplatte wurden mit dem Spatel einige Gramm der zu messenden Paste luftblasenfrei aufgetragen. Dabei wurde darauf geachtet, dass nach dem Zusammenfahren des Messsystems etwas Paste gleichmäßig aus dem Messsystem herausquellen konnte (nicht mehr als 6 mm rundum). Der Überschuss wurde mittels Spatel entfernt. Anschließend wurde die Temperierhaube über der Probe positioniert und die Messung gestartet. Bestimmt wurde die komplexe Viskosität der Paste nach 24 Stunden (Lagerung der Paste bei 25 °C in einem Temperierschrank der Fa. Memmert) in Abhängigkeit von der Temperatur.

Als Maß für die Gelierung wurde ein deutlicher Anstieg der komplexen Viskosität betrachtet. Als Vergleichswert wurde daher die Temperatur bei Erreichen einer Pastenviskosität von 1000 Pa*s verwendet. Die erhaltenen Ergebnisse sind in Tabelle 5 aufgeführt.

**Tabelle 5: Gelierung der Plastisole nach 24 Stunden, Temperatur in °C bei Erreichen einer Pastenviskosität von 1000 Pa*s.**

| Rezeptur | Weichmacherzusammensetzung nach Tabellen 1-3 | Geliertemperatur [°C] |
|---|---|---|
| 1 | DINCH | 88,7 |
| 2* | DINCH / MbPe-Tc (50:50) (3:1) | 83,6 |
| 3* | DINCH / MbPe-Tc (50:50) (2:1) | 82,7 |
| 4* | DINCH / MbPe-Tc (50:50) (1:1) | 80,7 |
| 5* | DINCH / MbPe-Tc (50:50) (1:2) | 79,9 |
| 6 | DINCD | 85,2 |
| 7* | DINCD / MbPe-Tc (50:50) (3:1) | 82,2 |
| 8* | DINCD / MbPe-Tc (50:50) (2:1) | 81,2 |
| 9* | DINCD / MbPe-Tc (50:50) (1:1) | 80,2 |
| 10* | DINCD / MbPe-Tc (50:50) (1:2) | 79,0 |
| 11* | DINCH / MbPe-Tc (35:65) (3:1) | 83,4 |
| 12* | DINCH / MbPe-Tc (35:65) (2:1) | 82,5 |
| 13* | DINCH / MbPe-Tc (35:65) (1:1) | 80,5 |
| 14* | DINCH / MbPe-Tc (35:65) (1:2) | 79,3 |
| 15* | DINCD / MbPe-Tc (35:65) (3:1) | 82,0 |
| 16* | DINCD / MbPe-Tc (35:65) (2:1) | 80,9 |
| 17* | DINCD / MbPe-Tc (35:65) (1:1) | 79,9 |
| 18* | DINCD / MbPe-Tc (35:65) (1:2) | 78,4 |
| 19* | DINCH / MbPe-Tc (7:93) (3:1) | 83,4 |
| 20* | DINCH / MbPe-Tc (7:93) (2:1) | 82,5 |
| 21* | DINCH / MbPe-Tc (7:93) (1:1) | 80,5 |
| 22* | DINCH / MbPe-Tc (7:93) (1:2) | 79,3 |
| 23* | DINCD / MbPe-Tc (7:93) (3:1) | 82,0 |
| 24* | DINCD / MbPe-Tc (7:93) (2:1) | 81,2 |
| 25* | DINCD / MbPe-Tc (7:93) (1:1) | 79,9 |
| 26* | DINCD / MbPe-Tc (7:93) (1:2) | 78,4 |

| | | |
|---|---|---|
| * Versuch mit erfindungsgemäßer Weichmacherzusammensetzung | | |

Durch eine Steigerung des MbPe-Tc-Anteils, unabhängig von der MbPe-Zusammensetzung, wird die Geliertemperatur im Vergleich zu den Primärweichmachen DINCH und DINCD deutlich reduziert. Des Weiteren wird ersichtlich, dass durch einen höheren n-Pentyl-Gehalt die Geliertemperatur nochmals begünstigt wird.

### Beispiel 4 - Herstellung von Folien

Die im Beispiel 2 hergestellten Plastisole wurden jeweils zu 1 mm dicken Folien verarbeitet. Dazu wurde zunächst Hochglanztrennpapier (Fa. Sappi, Italien) auf eine Größe von 30 x 44 cm zugeschnitten und im Spannrahmen der Streicheinrichtung LTSV für den Mathis-Ofen eingelegt. Danach wurde der Spannrahmen auf den Führungsrahmen aufgelegt, der Mathis-Ofen (Typ LTF) auf 200 °C eingestellt und der Rahmen nach Erreichen dieser Temperatur 15 Sekunden vorgewärmt. Danach wurde die Rakel in die Einspannvorrichtung gelegt und der Rakelspalt über Vorversuche so eingestellt, dass die Foliendicke nach Abschluss der Gelierung 1 mm (+/- 0,05 mm) betrug. Auf den vorderen Rand des Papiers wurde ein Klebestreifen angebracht, um überschüssiges Plastisol aufzufangen. Danach wurde das Plastisol vor der Rakel aufgetragen und diese durch Ziehen des Führungsrahmens mit der Rakel über das eingespannte Trennpapier verstrichen (3 m/min Geschwindigkeit). Danach wurde die Rakel herausgenommen und der Klebestreifen mit dem überschüssigen Plastisol abgenommen. Anschließend wurde der Spannrahmen in den Ofen eingefahren. Nach der Gelierung (2 Minuten bei 200 °C) wurde der Rahmen wieder aus dem Ofen herausgefahren und nach Abkühlen die Folie von dem Papier abgezogen.

### Beispiel 5 - Bestimmung des Masseverlusts (Aktivkohle-Verfahren)

In Anlehnung an DIN EN ISO 176 (Verfahren B) wurden aus den hergestellten PVC- Folien jeweils drei kreisrunde Scheiben mit einem Durchmesser von 5 cm ausgestanzt und zunächst für 16 Stunden im Exsikkator bei Raumtemperatur konditioniert. Auf der Analysenwaage wurden die Proben-Kreise ausgewogen, in einen Drahtkorb gelegt, welcher mit einer Klammer verschlossen wurde. Die Drahtkörbe wurden anschließend in eine mit Aktivkohle gefüllte Weißblechdose so platziert, dass sich zwischen den einzelnen Körben bzw. Korb und Deckel (gelocht) bzw. Boden jeweils etwa 130 ml Aktivkohle befanden.

Die befüllten Weißblechdosen wurden im vortemperierten Heizschrank (120 °C) so platziert, dass der Lüfter im Schrank nicht zugestellt wurde und die Dosen sich nicht berührten. Luftwechsel und Abluftklappe wurden auf jeweils 10 % eingestellt. Nach 72 Stunden wurden die Dosen wieder aus dem Schrank entnommen, abgekühlt, danach die einzelnen Proben den Körben entnommen, im Exsikkator nochmals 16 Stunden konditioniert und danach auf der Analysenwaage zurückgewogen. Die erhaltene Massedifferenz entsteht durch den Verlust an Weichmacher. Von den jeweils drei Massedifferenzen wurde der Mittelwert gebildet und prozentual der Verlust an Weichmacher errechnet. In Tabelle 6 sind die Ergebnisse der Flüchtigkeiten aufgeführt.

**Tabelle 6: Massenverlust der Prüfkörper nach drei Tagen bei 120 °C.**

| Foliennr. | Weichmacherzusammensetzung nach Tabellen 1-3 | Massenverlust nach 3 Tagen in Massen% |
|---|---|---|
| 1 | DINCH | -5,01 |
| 2* | DINCH / MbPe-Tc (50:50) (3:1) | -4,28 |
| 3* | DINCH / MbPe-Tc (50:50) (2:1) | -3,64 |
| 4* | DINCH / MbPe-Tc (50:50) (1:1) | -3,57 |
| 5* | DINCH / MbPe-Tc (50:50) (1:2) | -2,70 |
| 6 | DINCD | -3,10 |
| 7* | DINCD / MbPe-Tc (50:50) (3:1) | -2,84 |
| 8* | DINCD / MbPe-Tc (50:50) (2:1) | -2,56 |
| 9* | DINCD / MbPe-Tc (50:50) (1:1) | -2,40 |
| 10* | DINCD / MbPe-Tc (50:50) (1:2) | -2,10 |
| 11* | DINCH / MbPe-Tc (35:65) (3:1) | -4,62 |
| 12* | DINCH / MbPe-Tc (35:65) (2:1) | -3,96 |
| 13* | DINCH / MbPe-Tc (35:65) (1:1) | -3,71 |
| 14* | DINCH / MbPe-Tc (35:65) (1:2) | -3,05 |
| 15* | DINCD / MbPe-Tc (35:65) (3:1) | -2,94 |
| 16* | DINCD / MbPe-Tc (35:65) (2:1) | -2,76 |
| 17* | DINCD / MbPe-Tc (35:65) (1:1) | -2,43 |
| 18* | DINCD / MbPe-Tc (35:65) (1:2) | -2,14 |
| 19* | DINCH / MbPe-Tc (7:93) (3:1) | -4,73 |
| 20* | DINCH / MbPe-Tc (7:93) (2:1) | -4,00 |
| 21* | DINCH / MbPe-Tc (7:93) (1:1) | -3,79 |
| 22* | DINCH / MbPe-Tc (7:93) (1:2) | -3,08 |
| 23* | DINCD / MbPe-Tc (7:93) (3:1) | -2,78 |
| 24* | DINCD / MbPe-Tc (7:93) (2:1) | -2,66 |
| 25* | DINCD / MbPe-Tc (7:93) (1:1) | -2,42 |
| 26* | DINCD / MbPe-Tc (7:93) (1:2) | -1,92 |

| | | |
|---|---|---|
| * = erfindungsgemäße Zusammensetzung | | |

Durch eine Steigerung des MbPe-Tc-Anteils wird die Flüchtigkeit der Plastisole verringert. Bei den Verbindungen nach Formel (1) mit hohem n-Pentanolgehalt kann die Flüchtigkeit noch stärker abgesenkt werden.

### Beispiel 6 - Bestimmung der Shore A Härte

Aus den nach Beispiel 4 hergestellten Folien wurden 4,8 x 4,8 cm² große Stücke geschnitten. Diese wurden in einen Pressrahmen zu 6-8 Lagen, je nach Folienstärke, gestapelt und bei 170 °C und 200 bar verpresst. Hiernach wurden die Prüfkörper für mind. 16 h im Klimaraum unter Normklima (25°C, 50 % Raumfeuchte) konditioniert und anschließend in Anlehnung an DIN 48-4 mit einem Shore-A-Messgerät der Fa. Zwick-Roell vermessen. Dabei wurden die Messwerte nach 3 Sekunden abgelesen. An jedem Prüfkörper wurden Messungen an drei verschiedenen Stellen durchgeführt und der Mittelwert gebildet. Die Ergebnisse sind in Tabelle 7 aufgeführt.

**Tabelle 7: Shore A Härten der gepressten Prüfkörper.**

| Rezeptur | Weichmacherzusammensetzung nach Tabelle 1-3 | Shore A |
|---|---|---|
| 1 | DINCH | 86.0 |
| 2* | DINCH / MbPe-Tc (50:50) (3:1) | 83.7 |
| 3* | DINCH / MbPe-Tc (50:50) (2:1) | 82.8 |
| 4* | DINCH / MbPe-Tc (50:50) (1:1) | 81.7 |
| 5* | DINCH / MbPe-Tc (50:50) (1:2) | 81.0 |
| 6 | DINCD | 83.6 |
| 7* | DINCD / MbPe-Tc (50:50) (3:1) | 82.2 |
| 8* | DINCD / MbPe-Tc (50:50) (2:1) | 81.6 |
| 9* | DINCD / MbPe-Tc (50:50) (1:1) | 81.0 |
| 10* | DINCD / MbPe-Tc (50:50) (1:2) | 80.2 |
| 11* | DINCH / MbPe-Tc (35:65) (3:1) | 83.8 |
| 12* | DINCH / MbPe-Tc (35:65) (2:1) | 83.3 |
| 13* | DINCH / MbPe-Tc (35:65) (1:1) | 81.9 |
| 14* | DINCH / MbPe-Tc (35:65) (1:2) | 81.2 |
| 15* | DINCD / MbPe-Tc (35:65) (3:1) | 82.7 |
| 16* | DINCD / MbPe-Tc (35:65) (2:1) | 82.0 |
| 17* | DINCD / MbPe-Tc (35:65) (1:1) | 81.5 |
| 18* | DINCD / MbPe-Tc (35:65) (1:2) | 80.6 |
| 19* | DINCH / MbPe-Tc (7:93) (3:1) | 83.1 |
| 20* | DINCH / MbPe-Tc (7:93) (2:1) | 82.3 |
| 21* | DINCH / MbPe-Tc (7:93) (1:1) | 80.8 |
| 22* | DINCH / MbPe-Tc (7:93) (1:2) | 80.3 |
| 23* | DINCD / MbPe-Tc (7:93) (3:1) | 82.0 |
| 24* | DINCD / MbPe-Tc (7:93) (2:1) | 81.4 |
| 25* | DINCD / MbPe-Tc (7:93) (1:1) | 80.5 |
| 26* | DINCD / MbPe-Tc (7:93) (1:2) | 79.5 |

| | | |
|---|---|---|
| * = erfindungsgemäße Zusammensetzung | | |

Die Effizienz gemessen anhand einer sinkenden Shore A Härte wird durch die Beimischung der MbPe-Tc-Ester begünstigt.

## Patentansprüche

1. Weichmacherzusammensetzung enthaltend eine Verbindung der Formel (1) 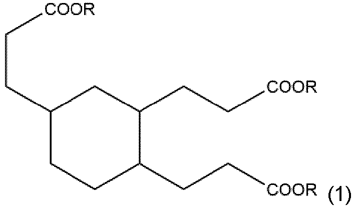 bei denen die beiden Alkylgruppen R jeweils 5 Kohlenstoffatome aufweisen von denen 45 bis 97 Mol% n-Pentylreste, vorzugsweise 55 bis 97 Mol% n-Pentylreste sind und mindestens einen 1,2- oder 1,4-Dialkylcyclohexandicarbonsäureester, wobei die beiden Alkylgruppen des 1,2- oder 1,4-Dialkylcyclohexandicarbonsäureester jeweils 8, 9 oder 10 Kohlenstoffatome aufweisen.

2. Weichmacherzusammensetzung nach Anspruch 1, wobei die beiden Alkylgruppen des 1,2- oder 1,4-Dialkylcyclohexandicarbonsäureester jeweils unabhängig voneinander aus der Gruppe, bestehend aus einer normalen Octylgruppe, einer Isooctylgruppe, einer 2-Ethylhexylgruppe, einer normalen Nonylgruppe, einer Isononylgruppe, einer n-Decylgruppe, einer Isodecylgruppe und einer 2-Propylheptylgruppe ausgewählt werden.

3. Weichmacherzusammensetzung nach Anspruch 1 oder 2, wobei die beiden Alkylgruppen des 1,2- oder 1,4-Dialkylcyclohexandicarbonsäureesters die gleiche Anzahl an Kohlenstoffatomen aufweisen.

4. Weichmacherzusammensetzung nach einem der vorhergehenden Ansprüche, wobei der 1,4-Dialkylcyclohexandicarbonsäureester der 1,4-Di-isononyl-cyclohexandicarbonsäureester (1,4-DINCH bzw. DINCD) oder der 1,4- Di-2-ethylhexylcyclohexandicarbonsäureester (1,4-DEHCH) und/oder der 1,2-Dialkylcyclohexandicarbonsäureester der 1,2-Di-isononyl-cyclohexandicarbonsäureester (DINCH) oder der 1,2-Di-2-ethylhexylcyclohexandicarbonsäureester (DEHCH) ist.

5. Weichmacherzusammensetzung nach einem der vorhergehenden Ansprüche, wobei die Verbindung nach Formel (1) und der 1,2- oder 1,4-Dialkylcyclohexandicarbonsäureester in einem Gewichtsverhältnis (Verbindung nach Formel (1) : 1,2- oder 1,4-Dialkylcyclohexandicarbonsäureester) von 1 : 99 bis 99 : 1, vorzugsweise 10 : 90 bis 90 : 10, besonders bevorzugt 20 : 80 bis 80 : 20 in der Weichmacherzusammensetzung vorhanden sind.

6. Weichmacherzusammensetzung nach einem der vorhergehenden Ansprüche, wobei die Weichmacherzusammensetzung zusätzlich ein epoxidiertes Öl oder einen epoxidierten Alkylester einer Fettsäure enthält.

7. Weichmacherzusammensetzung nach Anspruch 6, wobei das epoxidierte Öl aus der Gruppe bestehend aus epoxidiertem Sojabohnenöl, epoxidiertem Rizinusöl, epoxidiertem Leinöl, epoxidiertem Palmöl, epoxidiertem Stearat, epoxidiertem Oleat, epoxidiertem Tallöl, epoxidiertem Linoleat und Mischungen davon ausgewählt wird.

8. Weichmacherzusammensetzung nach Anspruch 7, wobei das epoxidierte Öl epoxidiertes Sojabohnenöl oder epoxidiertes Leinöl, vorzugsweise epoxidiertes Sojabohnenöl ist.

9. Weichmacherzusammensetzung nach einem der Ansprüche 6 bis 8, wobei das epoxidierte Öl in einer Menge von 1 bis 150 Gewichtsteilen bezogen auf 100 Gewichtsteile der Gesamtsumme aus der Verbindung gemäß Formel (1) und dem 1,2- oder 1,4-Dialkylcyclohexandicarbonsäureester in der Weichmacherzusammensetzung vorhanden ist.

10. Weichmacherzusammensetzung nach einem der vorherigen Ansprüche, wobei die Weichmacherzusammensetzung Mischester der Verbindung der Formel (1) umfasst, wobei mindestens 75 Mol% der Pentylreste und maximal 97 Mol% der Pentylreste im Mischester n-Pentylreste sind.

11. Kunststoffzusammensetzung, umfassend die Weichmacherzusammensetzung nach einem der Ansprüche 1 bis 10 und einen Kunststoff.

12. Kunststoffzusammensetzung nach Anspruch 11, wobei der Anteil der Weichmacherzusammensetzung 5 bis 150 Gewichtsteile pro 100 Gewichtsteile Kunststoff beträgt.

13. Kunststoffzusammensetzung nach Anspruch 11 oder 12, wobei der Kunststoff ein Polymer aus der Gruppe, bestehend aus Polyvinylchlorid (PVC), Polyalkylmethacrylat (PAMA), Polyvinylbutyral (PVB), Polyurethan, Polysulfide, Polymilchsäure (PLA), Polyhydroxybutyral (PHB), Nitrocellulose und Co-Polymeren von Vinylchlorid mit Vinylacetat oder mit Butylacrylat, ausgewählt wird.

14. Kunststoffzusammensetzung nach Anspruch 13, wobei der Kunststoff Polyvinylchlorid (PVC) ist.

15. Verwendung der Kunststoffzusammensetzung nach einem der Ansprüche 11 bis 13 in Klebstoffen, Dichtungsmassen, Beschichtungsmassen, Lacken, Farben, Plastisolen, Schäumen, Kunstleder, Fußbodenbelägen, insbesondere Deck- und Schaumschicht, Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, insbesondere in Schläuchen oder Blutbeuteln.
